**Europäisches Patentamt**

**European Patent Office**

**Office européen des brevets**

(19)

(11) Veröffentlichungsnummer: **0 344 279 B1**

(12) # EUROPÄISCHE PATENTSCHRIFT

(45) Veröffentlichungstag der Patentschrift :
**11.03.92 Patentblatt 92/11**

(51) Int. Cl.⁵ : **A61B 17/22, A61M 29/02,
A61M 25/00**

(21) Anmeldenummer : **89900572.2**

(22) Anmeldetag : **19.12.88**

(86) Internationale Anmeldenummer :
**PCT/DE88/00769**

(87) Internationale Veröffentlichungsnummer :
**WO 89/05609 29.06.89 Gazette 89/14**

(54) **BALLONKATHETER ZUM REKANALISIEREN VON STENOSEN IN KÖRPERKANÄLEN.**

(30) Priorität : **22.12.87 DE 3743578**

(43) Veröffentlichungstag der Anmeldung :
**06.12.89 Patentblatt 89/49**

(45) Bekanntmachung des Hinweises auf die
Patenterteilung :
**11.03.92 Patentblatt 92/11**

(84) Benannte Vertragsstaaten :
**AT BE CH DE FR GB IT LI LU NL SE**

(56) Entgegenhaltungen :
**EP-A- 0 193 831**

(56) Entgegenhaltungen :
**EP-A- 0 213 749
EP-A- 0 213 765
DE-A- 2 758 040
US-A- 4 643 186**

(73) Patentinhaber : **ZEIHER, Andreas
Klarastrasse 55
W-7800 Freiburg (DE)**

(72) Erfinder : **ZEIHER, Andreas
Klarastrasse 55
W-7800 Freiburg (DE)**

(74) Vertreter : **Rackette, Karl, Dipl.-Phys. Dr.-Ing
Kaiser-Joseph-Strasse 179 Postfach 1310
W-7800 Freiburg (DE)**

EP 0 344 279 B1

## Beschreibung

Die Erfindung betrifft einen Ballonkatheter zum Rekanalisieren von Stenosen in Körperkanälen, insbesondere Koronargefäßen und peripheren arteriellen Gefäßen, mit einem Ballon, der über einen in den Balloninnenraum mündenden Inflationstubus mit Hilfe eines Gases oder einer Flüssigkeit aufweitbar ist, sowie mit einer im Balloninnern angeordneten Mikrowellenantenne, die über ein Koaxialkabel mit einem Mikrowellengenerator verbindbar ist.

Ballonkatheter zum Rekanalisieren von Stenosen in Koronargefäßen werden bereits seit einiger Zeit von Kardiologen eingesetzt, wobei die Restenosierung mit bis zu 35 % der Anwendungen als wichtigste Spätkomplikation der klinisch außerordentlich bewährten Methode problematisch ist. Aus diesem Grunde wurde vorgeschlagen, mit Hilfe eines Lichtwellenleiters Laserlicht zur thermischen Nachbehandlung einer Ballondilatation zu verwenden (The American Journal of Cardiology, Vol. 56, Seite 953).

Ein Ballonkatheter der eingangs genannten Art ist in der US-A-4 643 186 beschrieben. Das Einführen eines derartigen Ballonkatheters in weit entfernt liegende verzweigte Gefäße, insbesondere Koronargefäße, erfordert ein großes Geschick. Zum leichteren Einführen von Ballonkathetern in Koronargefäßen ist es, wie sich aus der EP-A-0 213 749 ergibt, bereits bekannt, Führungsdrähte vorzusehen, die in ein Koronargefäß eingeführt werden, so daß ein mit einem Führungsschlauch versehener Ballonkatheter entlang dem Führungsdraht zur zu behandelnden Stenose vorgeführt werden kann. Es ist jedoch nicht möglich, einen Ballonkatheter gemäß der EP-A-0 213 749 mit einer aus der US-A-4 643 186 bekannten Mikrowellenantenne zu versehen, da der Führungsdraht Mikrowellen absorbieren würde und bei zurückgezogenem Führungsdraht Blut in den Führungsschlauch eindringen und dort bei eingeschaltetem Mikrowellengenerator koagulieren würde.

In der EP-A2 182 689 ist ein Ballonkatheter beschrieben, bei dem das den Ballon umgebende Gewebe mit Hilfe von in den Ballon eingespeistem Laserlicht erwärmt wird. Aus dieser Druckschrift ist es ebenfalls bekannt, ein elektrisches Heizelement in der Nähe des distalen Endes des Führungsdrahtes vorzusehen, das von dem Ballon umgeben ist und die im Ballon vorhandene Flüssigkeit aufheizt. Der sich durch den Ballon erstreckende Führungsschlauch hat einen Durchmesser, der nur geringfügig größer als der Durchmesser des Führungsdrahtes ist, um die Gefahr der Koagulation von in den Führungsschlauch eindringendem Blut zu vermeiden.

Aus Biomedizinische Technik Band 32, Ergänzungsband September 1987, Seiten 33 bis 36 ist es bekannt, Hochfrequenzenergie zur Stützung der Ballondilatation einzusetzen. Dabei wird vorgeschlagen, den Ballonkatheter mit einer bipolaren Elektrodenkonfiguration aus zwei Streifenelektroden zu versehen. Die Streifenelektroden werden an einem Generator mit einer Frequenz von 0,5 bis 1 MHz für eine Dauer von bis zu 38 Sekunden und eine Ausgangsenergie von maximal 50 Watt angeschlossen. Durch Hochfrequenzkoagulation im Bereich der Streifenelektroden soll bei Tierversuchen geklärt werden, ob eine stabilisierte Dilatation erreicht werden kann.

Eine Vorrichtung zur thermischen Behandlung des Körperhöhlen umgebenden Gewebes mit Temperatursteuereinrichtungen ist auch in der US-A 4 658 836 beschrieben, wobei eine direkte Erwärmung des Körpergewebes durch elektromagnetische Strahlung im Frequenzbereich von Radiowellen oder Mikrowellen erfolgt.

Der DE-C2 30 11 322 ist eine Strahlungssonde für eine Einrichtung zur Mikrowellenbehandlung von Körpergewebe zu entnehmen, die gleichzeitig die Anwendung einer Hyperthermie und einer Radiotherapiebehandlung gestattet, wobei das Gewebe mit Mikrowellenenergie bestrahlt wird. Zur Konzentration der Mikrowellenstrahlung auf den gewünschten Gewebebereich ist die in eine Körperhöhle einführbare Strahlungssonde mit einem Koaxialkabel ausgestattet, dessen unabgeschirmtes Ende in der Strahlungssonde unsymmetrisch von einem kolbenförmigen Körper umgeben ist.

Die US-A 4 662 383 beschreibt einen Ballonkatheter für die Hyperthermie von Tumoren, der über eine Zuführleitung mit einer Kühlflüssigkeit beaufschlagt wird, die über eine Abflußleitung aus dem Balloninnern abfließen kann. Im Innern des Ballons ist eine Mikrowellenantenne vorgesehen, die einen bis in das umgebende Gewebe hineinreichenden Strahlungsbereich hat. Als Kühlflüssigkeit sind Flüssigkeiten mit einem niedrigen Absorptionskoeffizienten für Mikrowellen vorgesehen, um das Eindringen der Mikrowellen in das Gewebe nicht zu behindern.

Ausgehend vom oben erörterten Stand der Technik liegt der Erfindung die Aufgabe zugrunde, einen Ballonkatheter der eingangs genannten Art zu schaffen, der entlang einem Führungsdraht vorgeschoben werden kann, ohne daß beim Einschalten des Mikrowellengenerators in den den Ballon durchquerenden Führungsschlauch eindringendes Blut koaguliert wird.

Diese Aufgabe wird erfindungsgemäß dadurch gelöst, daß sich durch den Ballon ein Zur Aufnahme eines Führungsdrahtes vorgesehener Führungsschlauch erstreckt, der aus einem leicht verformbaren Material besteht und dadurch bei dem zur Dilatation erforderlichen Balloninnendruck bei aus dem Bereich der Mikrowellenantenne zurückgezogenem Führungsdraht abdichtend abquetschbar ist.

Die Mikrowellenantenne im Balloninnern dient zunächst dazu, die zum Dilatieren der Ballonhülle verwendete Flüssigkeit aufzuheizen, bei der es sich um eine Mikrowellen gut absorbierende Flüssigkeit, beispielsweise ein Gemisch aus einer Kochsalzlösung und einem jodhaltigen Kontrastmittel handelt, dem gegebenenfalls andere Substanzen zur Erhöhung der Mikrowellenabsorption beigefügt sein können, wie beispielsweise Metallpartikel, die in der Flüssigkeit schweben. Die radial-symmetrische Ausbreitung der Mikrowellen führt zu einer homogenen radial-symmetrischen Erwärmung der Flüssigkeit.

Um ein direktes Eindringen von Mikrowellen in das den Ballonkatheter umgebende Gewebe zu verhindern, ist die Ballonhülle vorzugsweise mit einer Metallbeschichtung versehen. Um zu verhindern, daß in dem die Ballonhülle durchquerenden Führungsschlauch vorhandenes Blut beim Einschalten des Mickrowellengenerators koaguliert, ist der Führungsschlauch durch den Druck der zum Dilatieren verwendeten Flüssigkeit zusammenquetschbar, so daß darauf verzichtet werden kann, den Führungsschlauch mit einer abschirmenden Metallisierung zu umgeben, die im Innern des Ballons eine gleichmäßige Verteilung der Mikrowellenenergie stören würde.

Bei einem zweckmäßigen Ausführungsbeispiel der Erfindung besteht die Mikrowellenantenne aus dem vorderen, von seiner Abschirmung befreiten Ende eines Koaxialkabels. Dabei ist die Anordnung so getroffen, daß sich der metallische Innenleiter entlang der Mittellängsachse des aufgeweiteten Ballons erstreckt, um einen gleichmäßigen Abstand von der Ballonwand und somit eine gleichmäßige thermische Behandlung zu gestatten.

Zweckmäßige Ausgestaltungen und Weiterbildungen der Erfindung sind Gegenstand von Unteransprüchen.

Nachfolgend wird ein Ausführungsbeispiel der Erfindung anhand der Zeichnung näher erläutert. Es zeigen:

Fig. 1 einen Ballonkatheter gemäß der Erfindung im Längsschnitt,

Fig. 2 einen Ballonkatheter gemäß der Erfindung im aufgeweiteten Zustand, teilweise im Schnitt und teilweise in perspektivischer aufgeschnittener Darstellung und

Fig. 3 einen Querschnitt durch den mittleren Bereich der aufgeweiteten Ballonhülle des Ballonkatheters.

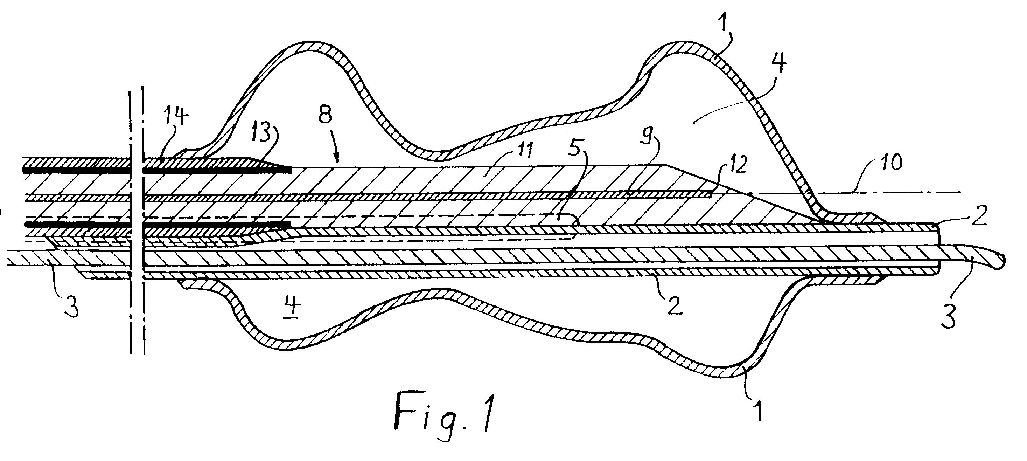

In Fig. 1 ist der Ballonkatheter zum Rekanalisieren von Stenosen in Koronargefäßen im Längsschnitt dargestellt. Der Ballonkatheter verfügt über einen Ballon aus einer Ballonhülle 1, die aus einem flexiblen hitzebeständigen Kunststoff besteht. Die Ballonhülle 1 ist außen und/oder innen mit einer in der Zeichnung nicht erkennbaren Metallbeschichtung versehen. Die metallische Ballonbeschichtung besteht vorzugsweise aus einem Gold-, Silber-, Chrom-, Chromnickel- oder Kupferfilm. Dabei kann die Beschichtung durchgehend gleich dick oder ein feines Netzwerk sein, das Unterbrechungen, beispielsweise in Form von Streifen aufweist. Die Beschichtung dient zum Abschirmen von in der Ballonhülle 1 erzeugten Mikrowellen nach außen sowie als durch die Mikrowellenabsorption aufgewärmte Heizfläche.

Die Ballonhülle 1 umgibt einen flexibel zusammendrückbaren Führungsschlauch 2, der je nach der Ausführungsform des Ballonkatheters nur geringfügig länger als die Ballonhülle ist. Der Führungsschlauch 2 dient dazu, den Ballonkatheter entlang einem in den Operationsbereich vorgeschobenen Führungsdraht 3 zu führen.

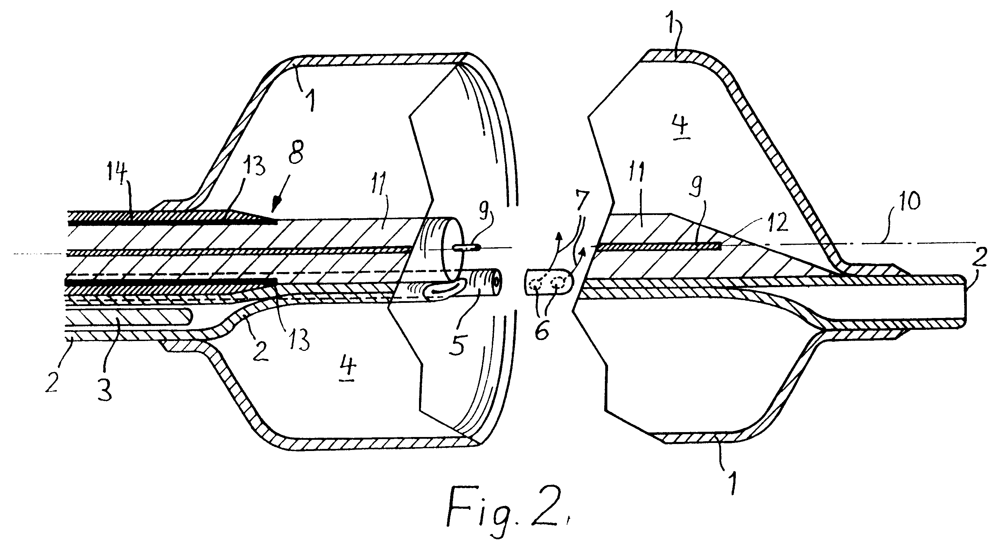

Zum Aufweiten der Ballonhülle 1 mit Hilfe einer in den Innenraum 4 der Ballonhülle 1 eingepreßten Flüssigkeit ist ein Inflationstubus 5 vorgesehen, der in Fig. 1 gestrichelt und in Fig. 2 teilweise perspektivisch dargestellt ist. Der Inflationstubus 5 weist an seinem vorderen Ende Öffnungen 6 auf, durch die eine Verbindung des Innenlumens des Inflationstubus 5 mit dem Innenraum 4 der Ballonhülle 1 geschaffen ist, so daß die Flüssigkeit entlang den Pfeilen 7 in den Innenraum 4 der Ballonhülle 1 eingedrückt werden kann. Wenn die Ballonhülle 1 des Ballonkatheters im Bereich der zu rekanalisierenden Stenose in einem Koronargefäß des Patienten liegt, wird der Führungsdraht 3 bis etwa in die in Fig. 2 erkennbare Stellung zurückgezogen.

Wie man in Fig. 2 erkannt, ist der Führungsschlauch an dem in den Fig. 1 und 2 linken Ende ausreichend lang bemessen, um zu verhindern, daß der Führungsdraht 3 beim Zurückziehen aus dem Eingriff des Führungsschlauches 2 gerät.

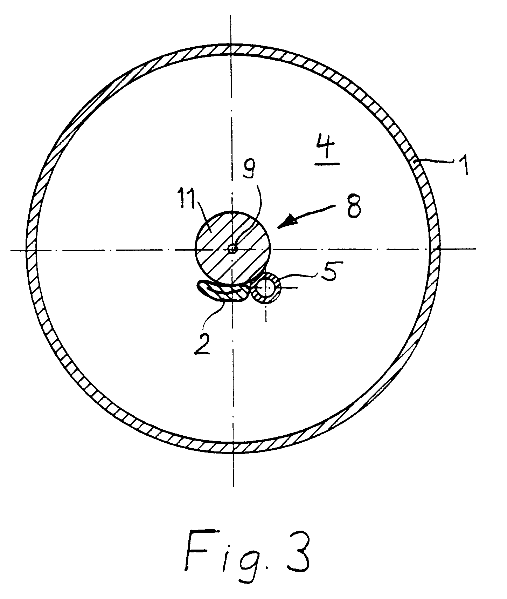

Wenn der Inneraum 4 der Ballonhülle 1 in der in Fig. 2 und Fig. 3 dargestellten Weise unter Druck mit der Flüssigkeit beaufschlagt wird, wird der Führungsschlauch 2 durch den Innendruck in der in der Ballonhülle 1 in den Fig. 2 und 3 dargestellten Weise zusammengequetscht, wodurch erreicht wird, daß kein durch Wärme koagulierendes Blut den Führungsschlauch 2 in Richtung zum zurückgezogenen Führungsdraht 3 durchströmt.

In den Fig. 1, 2 und 3 erkennt man oberhalb dem Führungsschlauch 2 und dem Inflationstubus 5 ein Koaxialkabel 8. Der metallische Innenleiter 9 des Koaxialkabels 8 erstreckt sich entlang der in den Fig. 1 und 2 eingezeichneten Mittellinie 10 bis in die Nähe der Ballonhülle 1, wobei jedoch zwischen dem in den Fig. 1 und 2 rechts dargestellten vorderen Ende des Innenleiters 9 und der aufgeweiteten Ballonhülle 1 ein Abstand besteht.

Der Innerleiter 9 des Koaxialkabels 8 ist von einer Isolierung 11 umgeben, die das vordere Ende 12 des Innenleiters 9 ebenfalls elektrisch von der im Innenraum 4 beispielsweise vorhandenen Flüssigkeit isoliert. Die Abschirmung 13 des Koaxialkabels 8 erstreckt sich um eine in etwa dem Abstand des vorderen Endes 12 des metallischen Innenleiters 9 von der Ballonhülle 1 entsprechenden Weg in den Innenraum 4 der Ballonhülle 1. In der bei Koaxialkabeln üblichen Weise ist die Abschirmung 13 des Koaxialkabels 8 von einem Außenschutzmantel 14 umgeben. Das Koaxialkabel 8 erstreckt sich gemeinsam mit dem Führungsdraht 3 und dem Inflationstubus 5 durch einen in der Zeichnung nicht dargestellten Führungskatheter, der beispielsweise im Leistenbereich des Patienten endet, bis zu einem Mickrowellengenerator mit einer Frequenz im Bereich von 400 MHz bis 10 GHz. Die Ausgangsleistung sowie die Einschaltdauern des Mikrowellengenerators sind voreinstellbar, wobei vorzugsweise ein in der Zeichnung nicht dargestellter Druckschalter den Innendruck in der Ballonhülle 1 erfaßt, um das Einschalten des Mickrowellengenerators nur bei einem Dilatationsdruck zu gestatten, bei dem der Führungsschlauch 2 abgequetscht ist und somit sicher eine Blutkoagulation durch die Mikrowellen im Führungsschlauch 2 vermieden wird. Insbesondere kann der Mikrowellengenerator so ausgebildet sein, daß die Hochfrequenzleistung impulsweise zur Verfügung gestellt wird und das Impulsprobenverhältnis sowie die Dauer eines Impulszugs den jeweiligen Operationsbedingungen entsprechend voreingestellt werden können.

Wenn die Ballonhülle 1 die in den Fig. 2 und 3 dargestellte etwa zylindrische Form angenommen hat und der Innenleiter 9 sich entlang der Mittelachse des aufzuweitenden Gefäßes befindet, wird durch Einschalten des Mikrowellengenerators die Flüssigkeit als Wärmereservoir um den als Antenne wirksamen Innenleiter 9 und anschließend durch Wärmeleitung die Gefäßinnenwand erhitzt, um eine thermische Nachbehandlung der mechanisch aufgeweiteten Gefäßwand, insbesondere eine Gefäßwandkoagulation durchzuführen und auf diese Weise die in die Gefäßwand eingebrachten Spannungen zu reduzieren, bzw. entstandene Lesionen zu koagulieren. Das Aufheizen der Flüssigkeit erfolgt aufgrund einer Absorption der Mikrowellenenergie. Um die Absorption zu erhöhen, ist es zweckmäßig als Flüssigkeit eine Kochsalzlösung zu verwenden, die mit einem jodhaltigen Röntgenkontrastmittel gemischt sein kann. Zusätzlich können auch Metallpartikel oder andere Mikrowellen absorbierende Stoffe beigemischt sein.

Die Wärmeeinflußzone läßt sich durch die Wahl der Flüssigkeit sowie durch die Frequenz der Mikrowellen, deren Leistung und Impulsform den jeweiligen Verhältnissen anpassen. Eine Anpassung ist auch durch die bereits erwähnte metallische Beschichtung auf der Ballonhülle 1 möglich. je nach der Wahl der Flüssigkeit und der Ausgestaltung der Beschichtung kann diese die Ballonhülle 1 nur abschirmen oder aber selbst auch aufgeheizt werden und die eigene oder aus der Flüssigkeit übertragene Wärme durch Wärmeleitung an die Umgebung abgeben.

Nach der Wärmebehandlung wird die Ballonhülle 1 dekomprimiert und der Ballonkatheter zusammen mit dem Führungsdraht 3 zurückgezogen.

Durch das Zurückziehen des Führungsdrahtes 3 vor dem Einschalten des Mickrowellengenerators wird auch erreicht, daß die im nicht abgeschirmten Bereich des Innenleiters 9 austretende Mikrowellenleistung nicht durch den Führungsdraht 3 abgeschirmt wird und insbesondere den Führungsdraht 3 nicht erwärmt.

Die Ballonhülle 1 ist an dem in Fig. 1 und 2 rechts dargestellten Ende mit dem Führungsschlauch 2 verschweißt. Am hinteren, in den Fig. 1 und 2 rechts dargestellten Ende ist die Ballonhülle 1 gemeinsam mit dem Führungsschlauch 2 und dem Außenschutzmantel 14 des Koaxialkabels 8 dicht verschweißt.

Bei dem oben beschriebenen mit Mikrowellen heizbaren Ballonkatheter wird Energie in den Ballon eingebracht und dort in der Flüssigkeit und/oder der Ballonhüllenbeschichtung in Wärme umgesetzt, ohne daß der Patient von einem Strom durchflossen wird und ohne daß ein galvanisch geschlossener Stromkreis vorliegt.

Der Ballonkatheter läßt sich besonders gut bei Koronargefäßen einsetzen. Es ist aber auch möglich, ihn bei entsprechend vergrößertem Durchmesser zum Aufweiten anderer arterieller Gefäße sowie beliebiger Körperkanäle und Körperhöhlen zu verwenden.

**Patentansprüche**

1. Ballonkatheter zum Rekanalisieren von Stenosen in Körperkanälen, insbesondere Koronargefäßen und peripheren arteriellen Gefäßen, mit einem Ballon (1), der über einen in den Balloninnenraum (4) mündenden Inflationstubus (5) mit Hilfe eines Gases oder einer Flüssigkeit aufweitbar ist, sowie mit einer im Balloninnern (4) angeordneten Mikrowellenantenne (9), die über ein Koaxialkabel (8) mit einem Mikrowellengenerator verbindbar ist, **dadurch gekennzeichnet**, daß sich durch den Ballon (1) ein Zur Aufnahme eines Führungsdrahtes (3) vorgesehener Führungsschlauch (2) erstreckt, der aus einem leicht verformbaren Material besteht und dadurch bei dem zur Dilatation erforderlichen Balloninnendruck bei aus dem Bereich der Mikrowellenantenne (9) zurückgezogenem Führungsdraht (3) abdichtend abquetschbar ist.

2. Ballonkatheter nach Anspruch 1, **dadurch gekennzeichnet**, daß die Mikrowellenantenne durch ein Koaxialkabel (8) gebildet ist, dessen Abschirmung (13) am im Balloninnern (4) liegenden vorderen Ende (9) entfernt ist und daß der metallische Innenleiter (9) des Koaxialkabels (8) im Innern (4) des Ballons (1) allseitig von einer Isolierung (11) umgeben ist.

3. Ballonkatheter nach einem der vorstehenden Ansprüche, **dadurch gekennzeichnet**, daß der nicht abgeschirmte Bereich des Innenleiters (9) gegenüber den beiden Enden des Ballons (1) zurückversetzt angeordnet ist.

4. Ballonkatheter nach einem der vorstehenden Ansprüche, **dadurch gekennzeichnet**, daß die Isolierung (11) des Koaxialkabels (8) am vorderen Ende in Richtung auf den Führungsschlauch (2) verjüngt ist.

5. Ballonkatheter nach einem der vorstehenden Ansprüche, **dadurch gekennzeichnet**, daß die Ballonhülle (1) auf ihrer Innenseite und/oder Außenseite mit einer Mikrowellen absorbierenden und/oder abschirmenden Beschichtung versehen ist.

6. Ballonkatheter nach Anspruch 5, **dadurch gekennzeichnet,** daß die Beschichtung eine Metallbeschichtung aus einem Gold-, Silber-, Chrom-, Chromnickel- oder Kupferfilm ist.

7. Ballonkatheter nach einem der vorstehenden Ansprüche, **dadurch gekennzeichnet**, daß die Leistung sowie die Einschaltdauer und Impulsform des Mikrowellengenerators einer Frequenz von 0,4 bis 10 GHz voreinstellbar sind.

8. Ballonkatheter nach einem der vorstehenden Ansprüche, **dadurch gekennzeichnet**, daß ein den Balloninnendruck erfassender Drucksensor vorgesehen ist, durch den der Mikrowellengenerator so steuerbar ist, daß ein Einschalten nur bei einem für ein Abquetschen des Führungsschlauches (2) ausreichenden Druck erfolgt.

9. Ballonkatheter nach einem der vorstehenden Ansprüche, **dadurch gekennzeichnet**, daß die Flüssigkeit zum Dilatieren der Ballonhülle (1) eine Mikrowellen gut absorbierende Flüssigkeit, insbesondere eine Kochsalz und/oder Jod enthaltende Lösung ist.


## Claims

1. A balloon catheter for dilating stenoses in body channels, in particular coronary vessels and peripheral arterial vessels, comprising a balloon (1) rich can be expanded by means of a gas or a liquid by way of an inflation tube (5) opening into the balloon interior (4), and a microwave antenna (9) which is arranged in the balloon interior (4) and rich can be connected to a microwave generator by way of a coaxial cable (8), characterised in that extending through the balloon (1) is a guide tube (2) rich is provided for receiving a guide wire (3) and comprises an easily deformable material and can thereby be sealingly squeezed off at the internal pressure in the balloon required for dilation, when the guide wire (3) is withdrawn from the region of the microwave antenna (9).

2. A balloon catheter according to claim 1 characterised in that the microwave antenna is formed by a coaxial cable (8), the shielding (13) of rich is removed at the front end (9) rich is disposed in the interior (4) of the balloon, and that the metal internal conductor (9) of the coaxial cable (8) is surrounded by an insulation (11) on all sides in the interior (4) of the balloon (1).

3. A balloon catheter according to one of the preceding claims characterised in that the unshielded region of the internal conductor (9) is arranged in set-back relationship relative to the two ends of the balloon (1).

4. A balloon catheter according to one of the preceding claims characterised in that the insulation (11) of the coaxial cable (8) is narrowed at the front end in the direction the the guide tube (2).

5. A balloon catheter according to one of the preceding claims characterised in that the balloon casing (1) is provided on its inward side and/or its outward side with a microwave-absorbing and/or shielding coating.

6. A balloon catheter according to claim 5 characterised in that the coating is a metal coating comprising a gold,silver, chromium, chrone-nickel or copper film.

7. A balloon catheter according to one of the preceding claims characterised in that the power as well as the switch-on time and pulse shape of the microwave generator at a frequency of from 0.4 to 10 GHz can be preset.

8. A balloon catheter according to one of the preceding claims characterised in that there is provided a pressure sensor rich detects the internal pressure in the balloon and by means of rich the microwave generator is so controllable that it is switched on only at a pressure rich is sufficient for the guide tube (2) to be squeezed off.

9. A balloon catheter according to one of the preceding claims characterised in that the liquid for dilation of the balloon casing (1) is a liquid rich provides good microwave absorption, in particular a solution containing common salt and/or iodine.

## Revendications

1. Cathéter gonflable d'élargissement de sténoses de canaux corporels, en particulier de vaisseaux coronaires et de vaisseaux artériels périphériques, comportant un ballon (1) qui peut être élargi avec un gaz ou un liquide au moyen d'un tube de gonflage (5) débouchant à l'intérieur (4) du ballon, ainsi qu'une antenne à micro-ondes (9), disposée à l'intérieur (4) du ballon, qui peut être reliée à un générateur de micro-ondes au moyen d'un câble coaxial (8), caractérisé en ce qu'un tube de guidage (2) prévu pour recevoir un fil conducteur (3) s'étend à travers le ballon (1), qui est constitué d'un matériau facilement déformable et, de ce fait, peut être écrasé, d'une manière étanche, à la pression interne du ballon nécessaire à la dilatation lorsque le fil conducteur (3) est retiré de la zone de l'antenne à micro-ondes (9).

2. Cathéter gonflable selon la revendication 1, caractérisé en ce que l'antenne à micro-ondes est formée par un câble coaxial (8) dont le blindage (13) est supprimé à l'extrémité avant 9 située à l'intérieur (4) du ballon, et que le conducteur intérieur métallique (9) du câble coaxial (8) est entouré de tous les côtés par une isolation (11) à l'intérieur (4) du ballon (1).

3. Cathéter gonflable selon l'une des revendications précédentes, caractérisé en ce que la zone non protégée du conducteur intérieur (9) est décalée en retrait par rapport aux deux extrémités du ballon (1).

4. Cathéter gonflable selon l'une des revendications précédentes, caractérisé en ce que l'isolation (11) du câble coaxial (8) est rétrécie à l'extrémité avant, en direction du tube de guidage (2).

5. Cathéter gonflable selon l'une des revendications précédentes, caractérisé en ce que l'enveloppe de ballon (1) est pourvue d'un revêtement absorbant et/ou empêchant les micro-ondes, sur sa face interne et/ou sur sa face externe.

6. Cathéter gonflable selon la revendication 5, caractérisé en ce que le revêtement est un revêtement métallique formé d'une mince couche d'or, d'argent, de chrome, de chrome-nickel ou de cuivre.

7. Cathéter gonflable selon l'une des revendications précédentes, caractérisé en ce que la puissance ainsi que la durée de mise en circuit et la forme d'onde d'impulsion du générateur de micro-ondes peuvent être préréglées à une fréquence de 0,4 à 10 GHz.

8. Cathéter gonflable selon l'une des revendications principales, caractérisé en ce qu'il est prévu un détecteur de pression captant la pression interne du ballon, au moyen duquel on peut commander le générateur de micro-ondes de manière telle qu'une mise en circuit s'effectue seulement à une pression suffisante pour écraser le tube de guidage (2).

9. Cathéter gonflable selon l'une des revendications précédentes, caractérisé en ce que le liquide de dilatation de l'enveloppe de ballon (1) est un liquide absorbant bien les micro-ondes, en particulier une solution contenant du chlorure de sodium et/ou de l'iode.

Fig. 1

EP 0 344 279 B1

Fig. 2.

Fig. 3